# EUROPÄISCHE PATENTSCHRIFT

(11) **EP 2 835 647 B1**
(45) Veröffentlichungstag und Bekanntmachung des Hinweises auf die Patenterteilung: **21.12.2016**
(21) Anmeldenummer: 14173350.1
(22) Anmeldetag: 20.06.2014
(51) Int. Cl.: G01N 33/569, A61L 2/20, A01N 63/00, G01N 33/50, A62D 3/00, C12N 15/62, G01N 33/566, A61L 2/18

(54) **Verfahren und Vorrichtung zur Dekontamination eines Habitats**
Method and device for the decontamination of a habitat
Procédé et dispositif de décontamination d'un habitat

(30) Priorität: 20.06.2013 DE 102013106460
(43) Veröffentlichungstag der Anmeldung: 11.02.2015
(73) Patentinhaber: Airbus Defence and Space GmbH, 85521 Ottobrunn (DE); Airbus DS GmbH, 82024 Taufkirchen (DE)
(72) Erfinder: Hummel, Thomas, 88682 Salem (DE); Reidt, Ulrich, 34613 Schwalmstadt (DE); Friedberger, Alois, 85667 Oberpframmern (DE); Kübler, Ulrich, 88677 Markdorf (DE); Schwarzwälder, Achim, 88677 Markdorf (DE); Kern, Peter, 88682 Salem (DE); Fetter, Viktor, 73441 Bopfingen (DE)
(74) Vertreter: Kastel, Stefan

(56) Entgegenhaltungen:
- EP-A2- 1 952 828
- WO-A1-92/21917
- WO-A1-2012/068131
- WO-A2-2008/006035
- WO-A2-2012/148868
- US-A- 4 520 016
- US-A1- 2004 063 189
- US-A1- 2004 208 853
- US-A1- 2009 047 173
- YOUNG WOOK KIM ET AL: "An ALD aluminum oxide passivated Surface Acoustic Wave sensor for early biofilm detection", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, Bd. 163, Nr. 1, 7. Januar 2012 (2012-01-07), Seiten 136-145, XP028461528, ISSN: 0925-4005, DOI: 10.1016/J.SNB.2012.01.021 [gefunden am 2012-01-16]
- Anonymous: "NASA Plans Test Of 'Electronic Nose' On International Space Station -- ScienceDaily", , 23. November 2008 (2008-11-23), XP055159327, Gefunden im Internet: URL:http://www.sciencedaily.com/releases/2 008/11/081119164314.htm [gefunden am 2014-12-18]
- RYAN M A ET AL: "The JPL electronic nose: Monitoring air in the U.S. Lab on the International Space Station", SENSORS, 2010 IEEE, IEEE, PISCATAWAY, NJ, USA, 1. November 2010 (2010-11-01), Seiten 1242-1247, XP031978093, DOI: 10.1109/ICSENS.2010.5690607 ISBN: 978-1-4244-8170-5

## Beschreibung

Die Erfindung betrifft ein Verfahren zur Dekontamination eines Habitats.

Die Notwendigkeit eines mikrobiologischen Monitoring und Controlling besteht nicht nur in Lebensräumen, d.h. Habitaten, in denen multiresistente Bakterien wie beispielsweise MRSA auftreten, sondern zeigt sich gerade auch innerhalb von sensiblen geschlossenen Lebensräumen. Gerade geschlossene Habitate neigen, begründet z.B. durch nur geringe Dimensionen, zu einem biologischen Ungleichgewicht. Diese biologische Belastung kann zu einer Gefährdung von Personen, aber auch von Materialien führen.

Deshalb besteht die Notwendigkeit, solche Habitate regelmäßig auf Kontaminationsspezies zu überprüfen und bei Überschreiten einer kritischen Kontaminationskonzentration zu beseitigen, beispielsweise durch Desinfektion.

Die sogenannte "Scheuer-Wisch"-Methode zur Desinfektion, bei der Desinfektionsmittel durch Wischen verteilt werden, bringen jedoch einen hohen manuellen Aufwand mit sich und die Effizienz der Methode ist in schwer zugänglichen Bereichen sehr eingeschränkt oder sogar nicht vorhanden.

Zusätzlich benötigen solche klassischen Verfahren hohe Flüssigkeitsmengen und insbesondere aggressive und/oder korrosive Desinfektionsmittel, die gegebenenfalls ein aufwändiges Vorbereiten des zu reinigenden Raumes erfordern, beispielsweise indem die Räume von sensiblen Einrichtungsgegenständen freigeräumt werden.

Dies ist aufwändig, zeitintensiv und wenig effektiv.

Kim et al., Sensors and Actuators B: Chemical: International journal devoted to research and development of physical and chemical transducers, 163 (2012), 136, beschreibt eine Vorrichtung und ein Verfahren zum Detektieren eines unspezifischen Biofilms.

In WO 2012/068131 A1 und WO 2008/006035 A2 ist ein Verfahren zum Dekontaminieren einer Räumlichkeit durch ein spezifisch ausgewähltes Dekontaminationsmittel beschrieben, wobei die Kontaminationsspezies als bekannt vorausgesetzt wird.

US 2009/047173 A1 und US 2004/063189 A1 beschreiben jeweils ein spezifisches Dekontaminationsverfahren ohne Überprüfung des Erfolges des Dekontaminationsverfahrens.

In Science Daily - "Nasa plans tests of ,electronic nose' on international space station" und Ryan et al., IEEE Sensors 2010 Conference, 1242 ist jeweils eine Vorrichtung beschreiben, die eine spezielle Kontaminationsspezies in einem Habitat identifizieren kann.

WO 92/21917 A1 offenbart allgemein ein unspezifisches Dekontaminationsverfahren unter Verwendung von Ozon und UV-Strahlung.

US 2004/208853 A1 beschreibt ein Dekontaminationverfahren unter Verwendung von Bakteriophagen, wobei der zu dekontaminierende Bakterienstamm als bekannt vorausgesetzt wird.

US 4 520 016 A1 beschreibt ein Verfahren, bei dem ein Habitat spezifisch dekontaminiert wird, wobei die Kontaminationsspezies als bekannt vorausgesetzt wird.

Aufgabe der Erfindung ist es daher, ein verbessertes Verfahren zur Dekontamination von Habitaten vorzuschlagen, das die genannten Nachteile überwindet.

Diese Aufgabe wird durch ein Verfahren zur Dekontamination eines Habitats mit den Merkmalen des Anspruchs 1 gelöst.

Vorteilhafte Ausgestaltungen der Erfindung sind Gegenstand der Unteransprüche.

Ein Verfahren zur Dekontamination eines Habitats weist die folgenden Schritte auf:
a) spezifisches Identifizieren wenigstens einer Kontaminationsspezies in dem Habitat;
b) Auswählen eines auf die identifizierte Kontaminationsspezies spezifisch wirksamen Dekontaminationsmittels;
c) Verteilen des Dekontaminationsmittels in dem zu dekontaminierenden Habitat;
d) Analyse des Dekontaminationszustandes in dem Habitat.

Somit wird eine vollständige Prozesskette der Identifikation und Dekontamination bzw. Desinfektion bereitgestellt. Das Verfahren ermöglicht es, eine bestimmte spezifische Belastung durch eine Kontaminationsspezies, insbesondere eine mikrobiologische Belastung, zu identifizieren und anschließend ein exakt auf die identifizierte Kontaminationsspezies abgestimmtes Dekontaminationsmittel anzuwenden.

Damit wird nun nicht mehr wie in klassischen Verfahren möglichst breitbandig der Kontamination entgegengewirkt, sondern unter Zuhilfenahme eines schmalbandigen, insbesondere eines biologisch spezifischen Ansatzes, das natürliche Gleichgewicht innerhalb von Habitaten, insbesondere innerhalb geschlossener Habitate, nachhaltig wiederhergestellt.

Die spezifische Kontamination wird vorteilhaft exakt identifiziert.

Dabei werden in Schritt b) bei mehreren spezifisch wirksamen Dekontaminationsmitteln biologische Dekontaminationsmittel ausgewählt.

Als biologische Dekontaminationsmittel werden spezifische Bakteriophagen und/oder spezifische bakteriolytische Proteine, insbesondere spezifische bakteriolytische Fusionsproteine, insbesondere auf Basis von Bakteriophagenproteinen, als Dekontaminationsmittel bereitgestellt.

Beispielsweise können als bakteriolytische Proteine Lysozym, Lysostaphin, lytM, Proteasen und/oder Hydrolasen verwendet werden.

Vorzugsweise werden dazu die Bakteriophagen genetisch derart modifiziert, dass Transduktionsgene zum Transferieren bakterieller Virulenzfaktorgenen in das Bakteriophagen inaktiviert werden.

Dies wird bevorzugt durch spezifische Mutation, insbesondere durch Punktmutation, Deletion, Insertion, Inversion und/oder Rasterschubmutation, erreicht.

In bevorzugter Ausgestaltung werden Fusionsproteine mit den folgenden Schritten hergestellt:
A) Klonieren eines Fusionsproteingens;
B) Transformieren des Fusionsproteingens in einen niederen prokaryotischen oder eukaryotischen Organismus;
C) Exprimieren von Fusionsproteinen in dem Organismus;
D) Reinigen der Fusionsproteine,
wobei in Schritt a1) ein Bakteriophagenprotein-Gen, ein Linkerprotein-Gen und ein lytisches Protein-Gen ligiert werden.

Bevorzugt wird in Schritt A) die Ligation seriell durchgeführt, wobei insbesondere zuerst das Bakteriophagenprotein-Gen, insbesondere N-terminal, und dann das Dabei werden in Schritt b) bei mehreren spezifisch wirksamen Dekontaminationsmitteln biologische Dekontaminationsmittel ausgewählt.

In bevorzugter Ausgestaltung werden spezifische Bakteriophagen und/oder spezifische bakteriolytische Proteine, insbesondere spezifische bakteriolytische Fusionsproteine, insbesondere auf Basis von Bakteriophagenproteinen, als Dekontaminationsmittel bereitgestellt.

Beispielsweise können als bakteriolytische Proteine Lysozym, Lysostaphin, lytM, Proteasen und/oder Hydrolasen verwendet werden.

Vorzugsweise werden dazu die Bakteriophagen genetisch derart modifiziert, dass Transduktionsgene zum Transferieren bakterieller Virulenzfaktorgenen in das Bakteriophagen inaktiviert werden.

Dies wird bevorzugt durch spezifische Mutation, insbesondere durch Punktmutation, Deletion, Insertion, Inversion und/oder Rasterschubmutation, erreicht.

In bevorzugter Ausgestaltung werden Fusionsproteine mit den folgenden Schritten hergestellt:
A) Klonieren eines Fusionsproteingens;
B) Transformieren des Fusionsproteingens in einen niederen prokaryotischen oder eukaryotischen Organismus;
C) Exprimieren von Fusionsproteinen in dem Organismus;
D) Reinigen der Fusionsproteine,
wobei in Schritt a1) ein Bakteriophagenprotein-Gen, ein Linkerprotein-Gen und ein lytisches Protein-Gen ligiert werden.

Bevorzugt wird in Schritt A) die Ligation seriell durchgeführt, wobei insbesondere zuerst das Bakteriophagenprotein-Gen, insbesondere N-terminal, und dann das Besonders vorteilhaft werden unterschiedliche Bakteriophagenstämme und/oder eine Mischung aus Bakteriophagen und bakteriolytischen Proteinen, insbesondere Fusionsproteinen, bereitgestellt.

Vorzugsweise werden die chemischen Dekontaminationsmittel aus der Gruppe Aldehyde, Aldehydderivate, insbesondere Formaldehydderivate, Perverbindungen, insbesondere Peressigsäure, Phenole, Phenolderivate, Alkohole, Halogene, Halogenverbindungen, wasserstoffperoxidbasierte Dekontaminationsmittel, insbesondere Acetylhydroperoxid, bereitgestellt.

Bevorzugt wird vor Schritt d) eine Kontaminationskonzentrationsgrenze definiert und in Schritt d) der Dekontaminationszustandes in dem Habitat mit der Kontaminationskonzentrationsgrenze verglichen, wobei bei Überschreiten der Kontaminationskonzentrationsgrenze die Schritte a) bis d) wiederholt werden.

Vorteilhaft wird dabei das zuvor ausgewählte Dekontaminationsmittel wiederholt oder ein anderes auf die identifizierte Kontaminationsspezies spezifisch wirksames Dekontaminationsmittel ausgewählt.

Vorzugsweise wird das Dekontaminationsmittel in Lösung, insbesondere in Pufferlösung, oder als Suspension verteilt..

Alternativ kann das Dekontaminationsmittel in Schritt c) in trockenem Zustand verteilt werden.

Die Verteilung in trockenem Zustand erfolgt bevorzugt in Pulverform, insbesondere im Massenbereich von µg bis mg, mehr insbesondere durch einen Feststoff-Luft- bzw. Partikel-Luft-Aerosolgenerator.

Eine Dekontaminationsvorrichtung zur Dekontamination eines Habitats weist eine Identifizierungseinrichtung zum Identifizieren wenigstens einer Dekontaminationsspezies in einem Habitat, eine Auswahleinrichtung zum Airbus Defence and Space GmbH, et al

Besonders vorteilhaft werden unterschiedliche Bakteriophagenstämme und/oder eine Mischung aus Bakteriophagen und bakteriolytischen Proteinen, insbesondere Fusionsproteinen, bereitgestellt.

Als chemische Dekontaminationsmittel werden chemische Dekontaminationsmittel aus der Gruppe Aldehyde, Aldehydderivate, insbesondere Formaldehydderivate, Perverbindungen, insbesondere Peressigsäure, Phenole, Phenolderivate, Alkohole, Halogene, Halogenverbindungen, wasserstoffperoxidbasierte Dekontaminationsmittel, insbesondere Acetylhydroperoxid, bereitgestellt. Bevorzugt wird vor Schritt d) eine Kontaminationskonzentrationsgrenze definiert und in Schritt d) der Dekontaminationszustandes in dem Habitat mit der Kontaminationskonzentrationsgrenze verglichen, wobei bei Überschreiten der Kontaminationskonzentrationsgrenze die Schritte a) bis d) wiederholt werden. Vorteilhaft wird dabei das zuvor ausgewählte Dekontaminationsmittel wiederholt oder ein anderes auf die identifizierte Kontaminationsspezies spezifisch wirksames Dekontaminationsmittel ausgewählt.

Vorzugsweise wird das Dekontaminationsmittel in Lösung, insbesondere in Pufferlösung, oder als Suspension verteilt..

Alternativ kann das Dekontaminationsmittel in Schritt c) in trockenem Zustand verteilt werden.

Die Verteilung in trockenem Zustand erfolgt bevorzugt in Pulverform, insbesondere im Massenbereich von µg bis mg, mehr insbesondere durch einen Feststoff-Luft- bzw. Partikel-Luft-Aerosolgenerator.

Eine Dekontaminationsvorrichtung zur Dekontamination eines Habitats ist in Anspruch 13 definiert.

In bevorzugter Ausgestaltung wird eine Verteileinrichtung bereitgestellt, die zur Ausbringung des Dekontaminationsmittels in trockenem Zustand und/oder in Lösung oder als Suspension geeignet ist.

Vorteilhaft ist die Verteileinrichtung zum Verteilen des Dekontaminationsmittels im ml-Bereich ausgebildet.

Geeignete Verteileinrichtungen weisen beispielsweise Dispersionsdüsen, Einstoffdüsen, Zweistoffdüsen mit innerer oder äußerer Mischung, Rotationszerstäuber und/oder Ultraschallzerstäuber auf.

Bevorzugt ist, wenn die Düse geeignet ist, kleine Tropfendurchmesser zu generieren. Weiter bevorzugt generiert die Düse nur geringe Drücke und Geschwindigkeiten innerhalb und außerhalb der Düse, die auf die Bakteriophagen wirken. Zusätzlich ist bevorzugt, wenn die Verteileinrichtung pumpenfrei arbeiten kann.

Ein geeignetes Beispiel einer Verteileinrichtung weist wenigstens einen Membran-Behälter, der vorteilhaft als Fluidtank zum Speichern unterschiedlicher Dekontaminationsmittel und/oder Reinigungslösungen bereitgestellt ist, und einen Druckluftspeicher mit vorzugsweise einer Kapazität von drei bis sechs bar auf. Diese Kapazität wird bevorzugt durch einen Kompressor bzw. einen Verdichter realisiert.

Vorteilhaft gibt der Drucklufttank gleichmäßig Druckluft an die Düse, beispielsweise über wenigstens eine Ventil gesteuert, und den Membran-Behälter ab. In dem Membranbehälter wirkt die Druckluft vorzugsweise als Vorschubgas.

Bevorzugt ist, wenn zwischen den Verteilschritten des Dekontaminationsmittels die Verteileinrichtung gereinigt wird, beispielsweise durch eine Reinigungslösung, z.B. in Form von Wasser.

Bevorzugt werden nach der Dekontamination des Habitats als Dekontaminationsmittel verwendete Bakteriophagen aus dem Habitat entfernt.

Dabei wird bevorzugt wenigstens ein Filter, insbesondere wenigstens ein HEPA-Filter (high efficiency particulate airfilter), verwendet.

Vorzugsweise werden die Schritte a) bis d) vollautomatisch mit einer Dekontaminationsvorrichtung durchgeführt, die eine Identifizierungseinrichtung zum Identifizieren einer Kontaminationsspezies und die Verteileinrichtung aufweist.

Bevorzugt weist die Dekontaminationsvorrichtung eine Steuereinrichtung zum Steuern der einzelnen Bauteile zur Durchführung des oben beschriebenen Verfahrens auf.

Ein Ausführungsbeispiel der Erfindung wird nachfolgend anhand der beigefügten Zeichnungen näher erläutert. Darin zeigt:
- Fig. 1: eine Verfahrenskette zur Durchführung eines Verfahrens zur Identifikation und Dekontaminierung eines Habitats;
- Fig. 2: eine Darstellung eines Identifikationssystems;
- Fig. 3: eine schematische Darstellung der mikrobioziden Wirkung von Wasserstoffperoxid und Acetylhydroperoxid;
- Fig. 4: ein Flussdiagramm zur Durchführung des Identifikations- und Dekontaminations-Verfahrens;
- Fig. 5: eine Längsschnittansicht durch eine kommerziell erhältliche Verteileinrichtung zur Verteilung von Dekontaminationsmitteln;
- Fig. 6: die kommerziell erhältliche Verteileinrichtung aus Fig. 5 in perspektivischer Ansicht;
- Fig. 7: eine kommerziell erhältliche Düsenvorrichtung zur Verwendung in dem Dekontaminationsschritt;
- Fig. 8: eine Dekontaminationsvorrichtung;
- Fig. 9: einen konzeptionellen Versuchsaufbau zur Überprüfung der Wirksamkeit des Identifikations- und Dekontaminationsverfahrens;
- Fig. 10: eine Box, die als Habitat in dem Versuchsaufbau verwendet werden kann;
- Fig. 11: Geschlitzte PET-Rohre zur Anwendung in dem Versuchsaufbau; und
- Fig. 12: ein Fusionsprotein auf Basis von Bakteriophagenproteinen.

Die Notwendigkeit eines mikrobiologischen Monitoring und Controlling ergibt sich nicht nur durch eine aktuelle Debatte, ausgelöst durch z.B. multiresistente Bakterien 10, sondern zeigt sich gerade auch innerhalb von sensiblen geschlossenen Lebensräumen, sogenannten Habitaten 12. Gerade geschlossene Habitate 12 neigen, begründet beispielsweise durch zu geringe Dimensionen, zu einem biologischen Ungleichgewicht. Diese biologische Belastung kann zu einer Gefährdung von Personen, aber auch Materialien (Raumstation Mir) führen.

Die bislang verwendete "Scheuer-Wisch"-Methode zur Desinfektion oder andere klassische Verfahren haben folgende Nachteile:
- hoher manueller Aufwand;
- Effizienz in schwer zugänglichen Bereichen sehr eingeschränkt oder nicht vorhanden;
- Verbrauch von hohe Flüssigkeitsmengen;
- Verwendung aggressiver/korrosiver Mittel, die gegebenenfalls ein aufwändiges Vorbereiten des zu reinigen Raumes erfordern (Entfernen von sensiblen Einrichtungsgegenständen).

Die Idee eines Identifikations-Dekontaminationsverfahrens besteht darin, die gesamte Prozesskette der mikrobiologischen Identifikation und Dekontamination bzw. Desinfektion als ein System zu realisieren. Ziel ist ein Verfahren, welches in der Lage ist, eine bestimmte spezifische mikrobiologische Belastung als Kontaminationsspezies 14 zu identifizieren und anschließend ein exakt auf den identifizierten Mikroorganismus abgestimmtes Dekontaminationsmittel 16 zu verwenden.

Ziel ist es, nicht mehr möglichst breitbandig der biologischen Kontamination entgegenzuwirken, sondern unter Hilfenahme eines schmalbandigen, gegebenenfalls auch spezifischen biologischen Ansatzes das natürliche Gleichgewicht innerhalb geschlossener Habitate 12 nachhaltig wiederherzustellen. Die spezifische Kontamination wird mittels eines Identifikationssystems 25, z.B. einem auf Elektrochemie basierenden Sensorarray 18, exakt identifiziert.

Mit dieser gewonnen Information wird aus einer Dekontaminationsmittel-Datenbank 20 ein dazugehöriges biologisches Desinfektionsmittel ausgewählt und mit minimalen Umweltauswirkungen im Habitat 12 verteilt.

Gerade geschlossene Habitate 12 neigen, begründet z.B. durch zu geringe Dimensionen, zu einem biologischen Ungleichgewicht. Der Mensch kann nicht als alleiniger Organismus innerhalb eines Habitates 12 betrachtet werden, da er selbst eine Vielzahl von Mirkoorganismen in sich trägt. Dies hat zur Folge, dass Mikroorganismen den Menschen stets begleiten werden und das Habitat 12 mehr oder weniger auch einen Lebensraum für Mikroorganismen darstellt.

Finden nun z.B. Bakterien 10 besonders günstige Lebensbedingungen, dann muss mit einem anhaltenden Wachstum gerechnet werden. Bakterien 10 stoßen bestimmte Stoffwechselendprodukte aus, die in größeren Mengen den Menschen gesundheitlich beeinträchtigen können und das Habitat 12 selbst als Lebensraum gefährden können.

Der klassische Ansatz, solch einer Kontamination entgegenzuwirken und somit die Lebensdauer des Habitates 12 zu verlängern, wird mit chemischen bzw. strahlungsbasierten Dekontaminationsverfahren bzw. Desinfektionsverfahren durchgeführt. Hierbei haben sich vor allem UV-Strahlung, thermische Behandlungen, sowie Dekontaminationsmittel 16 auf Wasserstoffperoxidbasis, bzw. Peressigsäurebasis bewährt. Gerade die chemischen Verfahren haben sich im kommerziellen Markt aufgrund ihrer breitbandigen Wirksamkeit durchgesetzt. Jedoch sind z.B. die hohe Materialbelastung (Korrosion) und Sicherheitsrisiken ein Grund dafür, dass diese Verfahren nicht oder nur unter Inkaufnahme von Nachteilen innerhalb von sensiblen Systemen eingesetzt werden können.

Aus diesen Nachteilen abgeleitet ermöglicht das vorgestellte Identifikations- und Dekontaminationverfahren einen alternativen Ansatz.

Ziel ist es, nicht mehr möglichst breitbandig der biologischen Kontamination entgegenzuwirken, sondern unter Hilfenahme eines schmalbandigen, insbesondere auch spezifischen biologischen Ansatzes das natürliche Gleichgewicht innerhalb geschlossener Habitate 12 nachhaltig wiederherzustellen.

Die spezifische Kontamination wird mittels eines Identifikationssystems 25, z.B. eines elektro-chemisch-basierten Sensorarrays 18, exakt identifiziert. Mit dieser gewonnenen Information wird aus einer Datenbank 20 ein dazugehöriges biologisches Desinfektionsmittel ausgewählt und mit minimalen Umweltauswirkungen im Habitat 12 verteilt. Das biologische Desinfektionsmittel wird möglichst frühzeitig in die Wachstumskurve der mikrobiologischen Kontamination eingreifen und sich durch diese nachhaltig ausbreiten. Solange die biologische Kontamination sich nicht in ihrer Struktur verändert, z.B. durch Genmutation, wird ein Langzeitschutz im Habitatgleichgewicht aufrecht erhalten.

Das Verfahren stellt damit eine innovative Herangehensweise an die Kontaminationsproblematik dar, da es eine spezifische, schmalbandige und nachhaltige Identifikation und Dekontamination im Gegensatz zu den bisherigen klassischen Verfahren darstellt.

"Dekontamination" entspricht einer Keimabtötung um mindestens log5-Stufen von koloniebildenden Einheiten (KBE, engl. Colony forming unit, CFU), laut Definition des Robert-Koch-Instituts. Im Allgemeinen ist darunter eine vollständige Keimabtötung zu verstehen.

Unter Desinfektion versteht man eine Keimreduktion (cfu reduction), d.h. es wird lediglich die Anzahl der CFU verringert (keine vollständige Abtötung).

Die Idee des o.g. Verfahrens besteht darin, die gesamte Prozesskette der mikrobiologischen Identifikation und Dekontamination bzw. Desinfektion als ein gesamtes System realisieren. Ziel ist es, ein Verfahren zu entwickeln, welches in der Lage ist, eine bestimmte spezifische mikrobiologische Belastung zu identifizieren und anschließend ein exakt auf den identifizierten Mikroorganismus abgestimmtes Desinfektionsmittel bzw. Dekontaminationsmittel 16 auszuwählen. Bei Einsatz eines Bakteriophagen 21-basierten Desinfektionsmittels sollte unter anderem ein nachhaltiger Effekt realisiert werden.

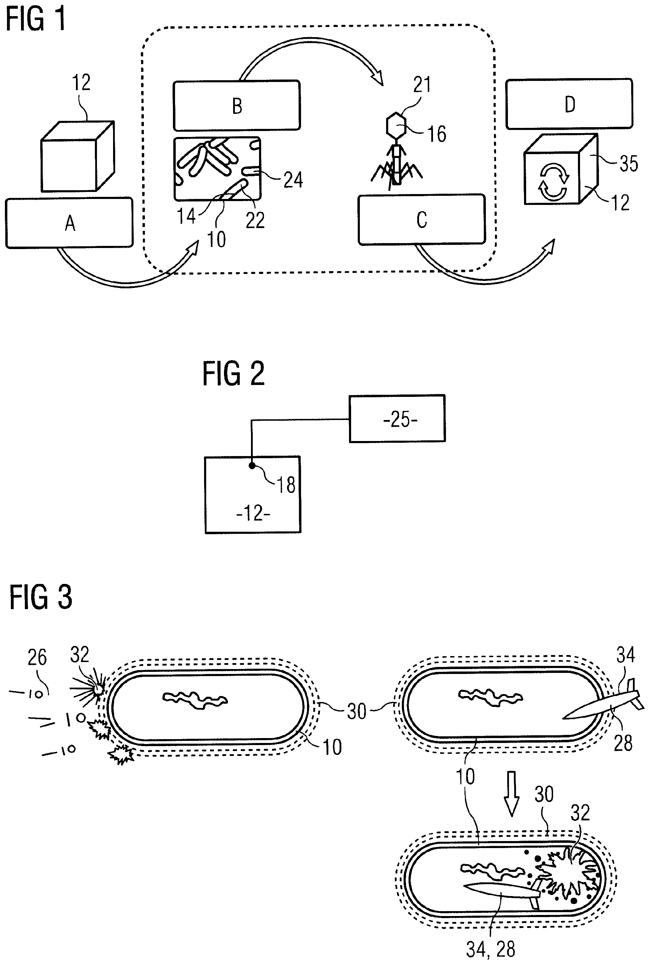

Fig. 1 zeigt eine Verfahrenskette des Identifikations- und Dekontaminationsverfahrens, wobei zunächst in Schritt A in einem Habitat 12 eine mikrobiologische Kontamination angenommen wird, dann in Schritt B die Kontaminationsspezies 14 bestimmt wird, dann zur Dekontamination Bakteriophagen 21 eingesetzt werden, und dann durch Analyse ermittelt wird, dass sich in dem Habitat 12 keine Kontaminationsspezies 14 mehr befindet.

Dies erfolgt aufgrund der negativen Auswirkungen von Mikroorganismen in Habitaten 12. Die Identifikation der spezifischen Mikroorganismen erfolgt beispielsweise durch ein Identifikationssystem, z.B. ein E-Nose-System (beispielsweise von Airsense).

Die biologisch spezifische Dekontamination mit Bakteriophagen 21 basiert auf Aerosolen, wobei als Backup ein chemisches Dekontaminationssystem bereitgestellt wird. Bakteriophagen 12 sind die natürlichen Feinde von Bakterien 10, so dass vorzugsweise nachhaltig ein biologisches Gleichgewicht erreicht werden kann.

Der erste Schritt - die Identifikation - der mikrobiologischen Belastung bildet die Grundlage einer spezifisch wirkenden Prozesskette. Spezifische Desinfektions- bzw. Dekontaminationsmittel 16 können vorteilhaft dann eingesetzt werden, wenn vorher eine exakte Bestimmung des Mikrobenstammes, d.h. des Bakterienstammes 22 durchgeführt wurde, um die Bakterienart 24 zu ermitteln.

Ein Verfahren zur Identifizierung von Bakterien kann der deutschen Patentanmeldung DE 10 2013 106 462.0 mit dem Titel "Detektionsverfahren zur Detektion von Bakterien, Verfahren zur Herstellung von Fusionsproteinen und Fusionsprotein", die als Anlage beigefügt ist, entnommen werden.

Ein Ziel dabei ist es, die Identifikationszeiten so zu verkürzen, dass man in wenigen Stunden, vorzugsweise in wenigen Minuten, in der Lage ist, die mikrobiologische Belastung vorzugsweise exakt zu benennen, um anschließend spezifische Gegenmaßnahmen einzuleiten.

Folgende Sensortypen werden kommerziell häufig eingesetzt:
- Metalloxide Semiconductor (MOS);
- Organic Conducting Polymers (CP);
- Quartz Crystal Microbalance (QCM);
- Surface Acoustic Wave (SAW);
- Amperometric Gas Sensors (AGS);
- Metaloxide Semiconductor Field-Effect Transistor (MOSFET).

Als eine mögliche Alternative wird in diesem Systemzusammenhang das in Fig. 2 gezeigte Identifikationssystem 25 vorgeschlagen.

Ein Sensorsystem wandelt Gasmischungen, die in ihrer spezifischen Zusammensetzung einen "Fingerabdruck" der jeweiligen Mikroorganismen aufweisen, in Signale um. Die technische Herausforderung ist die starke Abhängigkeit der Gaszusammensetzung von externen Umweltbedingungen, wie z.B. Temperatur, Luftfeuchte, Art und Stamm der Mikroben, Alter, Anzahl etc.

Für jede Mikrobenart und Stamm werden Versuche durchgeführt, die den olfaktorischen "Fingerabdruck" in einer Datenbank 20 sichern. Die aufgezeichneten elektrischen Signale können nun mit der Datenbank 20 verglichen werden und bekannte Arten und Stämme zeitnah bestimmt werden.

Das Erstellen dieser Datenbank 20 ist aufwendig und kostenintensiv und wird vorteilhaft von Fachpersonal durchgeführt. Die Datenbank 20 wird aber mit jedem neuen "Training" weiter wachsen und zu qualitativ besseren Ergebnissen führen. Sie kann aber auch im Sinne von wirtschaftlich sinnvollen Synergien für verschiedene Projekte bzw. Kunden genutzt werden.

Nachdem die spezifische mikrobiologische Belastung bekannt ist, stehen zwei Möglichkeiten zur Verfügung:
1. Handelt es sich bei der mikrobiologischen Belastung um Bakterien 10, dann kann das biologische Desinfektionsmittel via Bakteriophagen 21 eingesetzt werden.
   Eine detaillierte Beschreibung eines solchen Verfahrens ist der deutschen Patentanmeldung DE 10 2013 106 455.8 mit dem Titel "Verfahren zur Dekontamination von bakteriologischen Verunreinigungen", die als Anlage beigefügt ist, zu entnehmen.
2. Alternativ kann der Weg mit der klassischen Desinfektion beschritten werden. Gängige Desinfektionsmittel können in folgende Gruppen eingeteilt werden:
   - Formaldehyd bzw. Aldehyde bzw. deren Derivate;
   - Alkohole;
   - Perverbindungen;
   - Halogene;
   - Phenole bzw. deren Derivate;
   - Chlorverbindungen;
   - Sonstige Wirkstoffe.

Perverbindungen und Formaldehyde eigenen sich besonders für eine schnelle und breitbandige Anwendung. Beide Stoffe sind in der Lage, auch die wehrhaftesten Mirkoorganismen, z.B. sporenbildende Bakterien 10, effektiv zu desinfizieren. Häufig scheiden jedoch Formaldehyd-basierende Desinfektions- bzw. Dekontaminationsmittel 16 aus Gründen der Gesundheitsgefährdung für den Menschen aus.

Alternativ ist bevorzugt, wenn das Desinfektions- und Dekontaminationssystem mit einem gängigen chemischen Mittel betrieben werden kann, vorzugsweise mit Peressigsäure oder, wenn keine starke sporizide Wirksamkeit notwendig ist, mit einem Wasserstoffperoxid-basierten Desinfektions- bzw. Dekontaminationsmittel 16. Die Unterschiede bzgl. mikrobizider Wirksamkeit sind in Fig. 3 dargestellt.

Fig. 3 zeigt ein Schemabild der mikrobiziden Wirkung von Wasserstoffperoxid 26 und Acetylhydroperoxid 28. Die Bakterien 10 weisen einen Katalyseschutzschild 30 auf, der durch Aktivsauerstoff 32 deaktiviert wird, so dass
Wasserstoffperoxidmoleküle 26 bzw. Peressigsäuremoleküle 34 in das Bakterium 10 eindringen können.

Der Hauptwirkmechanismus der chemischen Dekontaminationsmittel 14 von Wasserstoffperoxid 26 und Peressigsäure 34 sind Aktiv-Sauerstoff-Atome, die in die Mikrobe eindringen und sie damit zum Platzen bringen. Je nach Konzentration von Wasserstoffperoxid 26 und Peressigsäure 34 kann eine Keimabtötung in gefordertem Umfang realisiert werden.

Die Vorteile von Wasserstoffperoxid 26 Peressigsäure 34-Verbindungen sind eine sehr schnelle Wirkdauer im Minutenbereich und die gute Kontrolle des Vorganges, da immer nur ein Aktiv-Sauerstoff-Atom z.B. ein Bakterium 10 eliminiert. Peressigsäure 34 -Verbindungen haben weiterhin den Vorteil, dass schwer durchdringbare Lipid-Moleküle, die meist in der Zellmembran von schwer zu eliminierenden Bakterien 10 auftreten, aufgebrochen werden können. Wasserstoffperoxid 26 hat dagegen den Vorteil, dass keine weiteren Rückstände auf den Oberflächen zurückbleiben außer Wasser, welches wie Kondenswasser über die Raumluft abgeführt werden kann.

Ein wesentlicher Nachteil der chemischen Wasserstoffperoxid 26- und Peressigsäure 34-Verbindungen ist die generell schwierige Materialverträglichkeit.
Aktiv-Sauerstoff 32 der der Hauptmechanismus der chemischen Dekontaminationsmittel 16 ist, fördert natürlich auch die Sauerstoffkorrosion an Metallen. Diese Korrosionsform ist nicht vermeidbar. Des Weiteren tritt zusätzlich Säurekorrosion an Metallen auf, da der pH-Wert Bereich z.B. von Peressigsäure 34 -Mitteln im sauren Bereich liegt und ohne Zugabe von Inhibitoren als Pufferlösungen hier starke Säurekorrosion auftritt. Auch zeigen nicht alle Kunststoffe gegenüber diesen Verbindungen hohe Verträglichkeiten.

Bei Einsatz von chemischen Dekontaminationsmitteln 16 sollte vorteilhaft auf die Materialverträglichkeit abgestimmt werden. Dies gilt auch bei Verwendungen von Inhibitoren, die den pH-Wert je nach Randbedingungen in den neutralen Bereich verschieben.

Damit einhergehend unterliegt der pH-Wert auf der Materialoberfläche großen Schwankungen. Dies wird vorteilhaft bei eventuellen Versuchen berücksichtigt, um Aussagen über den Verlauf der Säurekorrosion zu erhalten. Ein weiterer Nachteil ist, dass chemische Dekontaminationsmittel 16 nicht in der Lage sind, spezifisch gegen mikrobiologische Belastungen vorzugehen und somit immer in das gesamte Ökosystem eingreifen werden und alle Mikroben dekontaminieren oder desinfizieren, egal ob positiv für ein bestimmten Lebensraum (Habitat 12) oder negativ.

Vor allem technische Fragestellungen bzgl. Materialverträglichkeit gaben den Anstoß, Alternativen zu den bisherigen Desinfektions- bzw.
Dekontaminationsverfahren in Betracht zu ziehen. Der Ansatz einer spezifischen und biologischen Desinfektion hat insbesondere den Vorteil, dass nur sehr geringe Mengen an Fluid ausgebracht werden müssen oder gar keines, was wiederum äußerst positive Effekte auf die Materialverträglichkeit mit sich bringt. Der größte Vorteil von einem spezifisch wirkenden Desinfektionsmittel sind die minimalen Einflüsse auf die Umwelt. Unter Umwelt werden an dieser Stelle Menschen, Materialien, komplexe und sensible Systeme verstanden.

Nachfolgend sind die Vor- und Nachteile beider Möglichkeiten kurz zusammengefasst.

Die biologische Bakteriophagendesinfektion hat eine hohe Materialverträglichkeit, benötigt einen geringen bis sehr geringen (ca. 0,1 ml bis 20 I, abhängig von der Größe des Habitats 12) Flüssigkeitseinsatz, bringt einen nachhaltigen Schutz und weist in der Regel auch keine Gefährdung für den Menschen auf. Die Wirkung ist spezifisch, jedoch der technologische Aufwand größer als bei chemischen Dekontaminationsmitteln 16.

Chemische Dekontaminationsmittel 16 sind nicht oder nur teilweise verträglich, beispielsweise wegen Sauerstoffkorrosion, Säurekorrosion bei Metallen, und auch nicht alle kunststoffbeständig. Der Flüssigkeitseinsatz ist relativ hoch mit etwa 1 bis 2 Litern für 250 m³ und sie bringen keinen nachhaltigen Schutz. Während der Anwendung besteht Gefahr für den Menschen, so dass vorteilhaft Menschen in Habitat 12 evakuiert werden sollten. Sie weisen keine spezifische Wirkung auf, jedoch ist der technologische Aufwand geringer als bei biologischen Bakteriophagendesinfektionsverfahren.

Das Desinfektions- bzw. Dekontaminationsverfahren wird vorteilhaft in enger Zusammenarbeit mit dem Identifikationsverfahren optimale und spezifische Ergebnisse liefern. Dabei bringt eine kombinierte Variante zwischen biologischer Hauptdesinfektion und chemischer Volldekontamination, die im Fall von nicht vorhandenen Bakteriophagen 21 eingesetzt werden soll, eine gesamtheitliche Lösung.

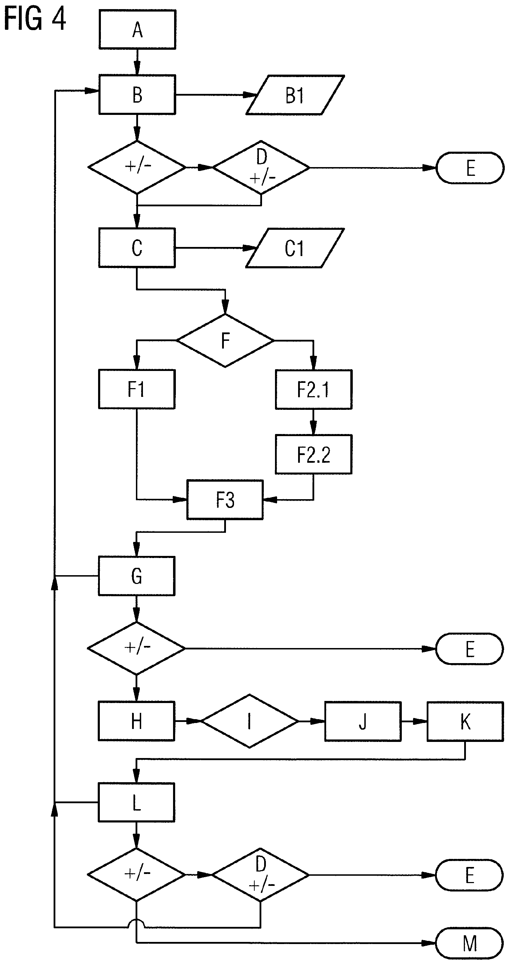

Ein Vorschlag für eine vollständige Prozesskette ist Fig. 4 dargestellt.

Im Schritt A wird angenommen, dass eine mikrobiologische Belastung vorliegt.

In Schritt B wird die Identifikation mit einem Identifikationssystem 25, wie z.B. einer elektronischen Nase, durchgeführt, wobei in Schritt B1 ein Vergleich mit einer mikrobiellen Datenbank 20 erfolgt. Wird ermittelt, dass keine mikrobielle Belastung vorliegt, kann optional Schritt D durchgeführt werden, in dem das Testsystem verändert wird. Liegt (Schritt E) keine mikrobiologische Belastung vor, endet das Verfahren an dieser Stelle.

Wird jedoch eine mikrobiologische Belastung identifiziert, wird in Schritt C ein Bakteriophagen 21-basiertes Desinfektionssystem angewendet, indem in Schritt C1 spezifische Bakteriophagen 21-Agenzien aus der Datenbank 20 ausgewählt werden.

Das Ausbringen der Bakteriophagen 21 erfolgt in Schritt F trocken oder als Fluid in einer Aerosol-Dispersion. Je nach trockener oder flüssiger Ausbringung wird bei trockener Ausbringung in Schritt F1 ein spezifisches Bakteriophagen 21-Pulver direkt dispergiert und in Schritt F2.1 bei flüssiger Ausbringung die Bakteriophagen 21 in einer Pulverlösung gelöst und dann in Schritt F2.2 diese Lösung dispergiert.

Es folgt Schritt F.3 "Warten" und dann Schritt G "Analyse" mit einem zweiten Prozessschritt, um festzustellen, ob es weiterhin eine mikrobiologische Belastung in dem Habitat 12 gibt. Ist dies nicht der Fall, folgt wieder Schritt E "Ende des Verfahrens".

Wird weiterhin eine mikrobiologische Belastung identifiziert, kommt es zu Schritt H "Anwendung eines Dekontaminationssystems", das auf chemischen Dekontaminationsmitteln 16 basiert ist. Dazu wird in Schritt I ein chemisches Agenzium, z.B. Wasserstoffperoxid 26 oder Peracetylsäure 24, ausgewählt und in Schritt J zu einem Fluid chemisch gelöst.

Es folgt in Schritt K die Dispersion des chemischen Fluides und in Schritt L eine Analyse des Habitats 12 mit einem dritten Prozessschritt, um festzustellen, ob es weiterhin eine mikrobielle Belastung in dem Habitat 12 gibt.

Ist dies nicht der Fall, kann optional eine andere Testmethode angewendet werden, wenn diese weiterhin keine mikrobielle Belastung nachweist, erfolgt Schritt E "Ende des Verfahrens".

Wird auch im dritten Analyseschritt eine weitere mikrobielle Belastung nachgewiesen, bedeutet dies, dass es sich um eine sehr widerstandsfähige Belastung handelt, die andere Agenzien zur Beseitigung benötigt (Schritt M).

In den Schritten G und L wird somit ein Dekontaminationszustand 35 des Habitats 12 ermittelt.

Bei dem Dekontaminationsansatz mittels Bakteriophagen 21 werden bevorzugt von Bakteriophagen 21 (Viren) angewendet, die spezifisch auf einen Bakterienstamm 22 wirken. Dies ist in der Regel gegeben, aber es ist nicht garantiert, dass diese Bakteriophagen 21 kommerziell (schon) verfügbar sind.

Die gesamte Desinfektion bzw. Dekontamination steht vorteilhaft im Zusammenspiel mit dem Identifikationssystem. Um gerade die Vorteile einer spezifischen und nachhaltigen Desinfektion zu nutzen, wird bevorzugt je nach Anwendungsfall die Bakteriophagen 21-gestützte Desinfektion angewendet. Hierbei erfolgt eine detaillierte Identifikation der Bakterienart 24 und Stammzugehörigkeit, damit mindestens eine spezifisch wirkende Bakteriophagenart ausgewählt werden kann. Es können auch zur Erhöhung der Effizienz mehrere wirksame Bakteriophagenarten gegen ein Bakterium 10 eingesetzt werden.

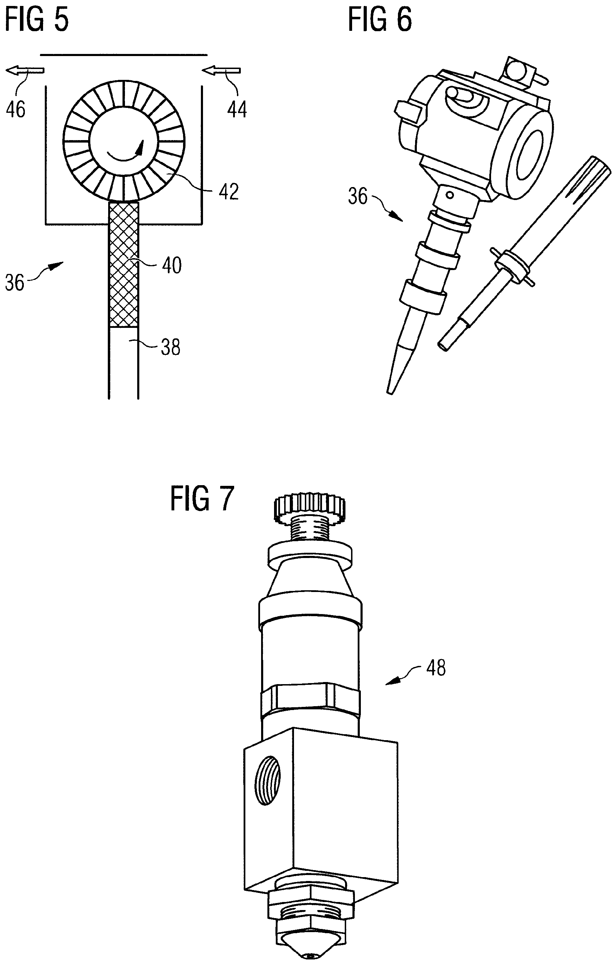

Die Bakteriophagenausbringung kann prinzipiell über zwei Möglichkeiten erfolgen:
1. Die trockene Ausbringung in Pulverform hat den Vorteil, dass keine Feuchtelasten auf die Umgebung ausgeübt werden (im Bereich der Elektronik keine Kurzschlüsse). Das Pulver, welches im mg-Bereich dosiert wird, wird vorteilhaft über einen Feststoff-Luft- oder Partikel-Luft-Aerosolgenerator ausgebracht. Diese "Partikelgeneratoren" arbeiten in der Regel mit größeren Mengen und über Schwerkraftabscheidemechanismen, die eine geregelte Dosierung ermöglichen. Sollte ein Feststoff-Luft-Aerosolgenerator im µg-Bereich (in-Orbit Anwendung) den Einsatz finden, dann empfiehlt sich ein System, welches mit einer Füllkammer und einem Vorschubkolben arbeitet. Fig. 5 zeigt eine kommerziell erhältliche Verteileinrichtung 36 zur trockenen Ausbringung von Aerosolen im Querschnitt. Sie weist zur Vorschubbewegung einen Vorschubkolben 38 auf. Über eine Staubbehälter 40 und eine Bürste 42 werden Luft 44 und Aerosol 46 gemischt und verteilt.

Fig. 6 zeigt die Außenansicht einer solchen kommerziell erhältlichen Verteileinrichtung 36.

Bei der trockenen Aerosolausbringung wird vorteilhaft eine gleichmäßige Verteilung innerhalb eines abgeschlossenen Systems mit sehr geringen Mikrogramm-Mengen realisiert. Dies kann beispielsweise im Beladungsverhältnis der Dispersionsdüse erfolgen. Das Beladungsverhältnis ist definiert mit Fluid bzw. Feststoff zu Luft 44 bzw. Treibgas. Es ist anzunehmen, dass, je höher der Treibgasanteil ist, desto feiner kann die Feststoffmenge nacheinander ausgebracht werden.

Weiter ist es vorteilhaft, wenn Bakteriophagen 21 gewählt werden, die effektiv durch die Verteilung an die Bakterien 10 andocken können, wenn sie im getrockneten Zustand vorliegen.

Vorzugsweise sollte eine Düsenanordnung gewählt werden, die einen möglichen Pulverstau weitgehend vermeidet.

2. Eine Fluid-gestützte Ausbringung hat den Vorteil, dass die Bakteriophagen 21 in einer Pufferlösung, die eine Nährlösung darstellen soll, gelöst werden. Die Menge der ausgebrachten Lösung liegt etwa im ml-Bereich. Der verfahrenstechnische Anspruch ist bei der Fluid-gestützten Ausbringung geringer als bei der trockenen Ausbringung, da viele Systeme, vorrangig Dispersionsdüsen, mit Zweistoffgemischen betrieben werden. Die Düsenauswahl dieser Fluid-Luft-Zerstäubung bedingt sich vor allem durch die Überlebensfähigkeit der Bakteriophagen 21. Diese werden vorteilhaft keinen extremen Geschwindigkeiten und Drücken innerhalb und außerhalb der Düse ausgesetzt. Weiterhin ist ein feiner Sprühnebel, also ein kleiner Tropfendurchmesser bevorzugt, um auch schlecht zugängliche Bereiche über die Raumluftströmung zu erreichen.

Nach einem Vergleich zwischen Rotationszerstäuber, Einstoffdüse, Zweistoffdüse und Ultraschallzerstäuber hat sich eine Zweistoffdüse mit innerer oder äußerer

Mischung, je nach gewünschter Sprühbreite und Tropfendurchmesser als relativ Bakteriophagenfreundlich ergeben. Kommerziell erhältlich sind Zweistoffdüsen unterschiedlicher Bauarten, auch im modularen Aufbau, so dass viele geeignete Kombinationen ausgewählt werden können.

Fig. 7 zeigt eine solche Zweistoffdüse 48.

Der generelle technische Anspruch für eine Fluid-gestützte Ausbringung ist vorteilhaft niedriger als bei der trockenen Ausbringung. Weiterhin ist die Effizienz der Desinfektion vorzugsweise höher, da die Bakteriophagen 21 im Fluid gelöst auf die Bakterien 10 treffen und somit ein erleichtertes Eindringen erfolgt.

Es ist bevorzugt, dass die Fluidförderung nicht durch Pumpen erfolgt, da sonst die gelösten Bakteriophagen 21 beschädigt werden könnten.

Nach der Bakteriophagen 21-gestützten Desinfektion wird bevorzugt eine erneute Identifikation bzw. Analyse des mikrobiotischen Zustandes durchgeführt, um eine Sicherstellung bzw. einen Nachweis zu erbringen, dass die mikrobielle Belastung unterhalb definierter Grenzen gebracht werden konnte.

Nach der Identifizierung wird die Dekontamination durchgeführt und danach die bereits angesprochene Verifizierung, um festzustellen, ob die Dekontamination erfolgreich war.

Sollte die Verifikation nach einer gewissen Wirkdauer des Desinfektionsmediums keine Veränderung von flüchtigen organischen Bestandteilen, die beispielsweise Stoffwechselendprodukte der Bakterien 10 darstellen, ergeben (z.B. Reduktion der Gasemissionen), so stehen folgende weitere Möglichkeiten zur Verfügung:
Die Durchführung eines weiteren biologischen Desinfektionsversuchs mit einem anderen Bakteriophagentyp oder Bakteriophagengemisch. Falls es sich z.B. nicht um Bakterien 10 handelt, sondern um eine Pilzart oder um Bakteriophagenresistente Bakterien 10, oder es existieren keine Bakteriophagen 21 gegen das detektierte Bakterium 10, dann wird bevorzugt eine klassische Fluid-gestützte chemische Dekontamination angewendet. Diese nicht-nachhaltige Behandlung wird aber breitbandig und mit den genannten Nachteilen erfolgen.

Zu den favorisierten Dekontaminationsmitteln zählen vor allem Wasserstoffperoxid 26 und Peressigsäure 34. Wasserstoffperoxid 26 hat den Vorteil, dass nur Wasser als Endprodukt zurückbleibt. Peressigsäure 34, welches eine Verestherung von Wasserstoffperoxid 26 ist, weist eine höhere mirkobiozide Wirksamkeit auf als Wasserstoffperoxid 26, hat aber den Nachteil, dass Natriumacetat und Wasser als Endprodukte zurückbleiben können. Bei erhöhter Feuchtigkeit kann das Salz Natriumacetat wieder in Lösung gehen und eine Säurekorrosion auf Metallen nach sich ziehen.

Sollten diese Dekontaminationsmittel 16 eingesetzt werden, dann werden sie bevorzugt je nach Dosierung mit einer spezifischen Menge Medium, z.B. Wasser, angemischt und ebenfalls bevorzugt über eine Dispersionsdüse fein im Raum verteilt. Da diese Mittel druckbeständig sind, werden keine spezifischen Anforderungen an die Düsenart gestellt. Lediglich ein kleiner Tropfendurchmesser bleibt anzustreben.

Um Synergien zwischen biologischer Verfahrenskette und chemischer Verfahrenskette zu realisieren, könnte durchaus der gleiche Düsentyp verwendet werden.

Es kann auch ein Zerstäuber, mit dem Bakterien 10 (z.B. Legionellen) in gelöster Form als Fluid in die Raumluft zerstäubt werden können, angewendet werden.

Innerhalb der bisherigen Betrachtungen konnten bevorzugte Eigenschaften einer Dekontaminationsvorrichtung 50 herausgearbeitet werden. Diese werden im folgenden Abschnitt kurz aufgeführt und zusammengefasst. Dabei wird die Identifikation an dieser Stelle nicht näher betrachtet, da vorausgesetzt wird, dass das Identifikationssystem 25 als eigenständiges System benutzt wird.

Aus den Betrachtungen der verfügbaren Desinfektionsverfahren und Dekontaminationsverfahren und deren Vor- und Nachteile können folgende bevorzugten Eigenschaften einer Dekontaminationsvorrichtung 50 definiert werden:
Die Dekontaminationsvorrichtung 50 sollte bevorzugt im Rahmen des technisch Möglichen ein Habitat 12 von einer biologischen Kontamination befreien können. Sie sollte vorteilhaft dazu ausgelegt sein, spezifisch zu desinfizieren, um gezielt einzelne Belastungen zu reduzieren. Sollte eine spezifische Desinfektion nicht erwünscht sein oder durchgeführt werden, sollte die Dekontaminationsvorrichtung 50 vorteilhaft ein Sekundärverfahren durchführen können, um die Kontamination zu eliminieren. Bevorzugt ist, wenn die Dekontaminationsvorrichtung 50 gereinigt werden kann, so dass unterschiedliche Lösungen, Desinfektionsmittel und Dekontaminationsmittel 16 in der gewünschten Qualität ausgebracht werden können. Zusätzlich ist bevorzugt, wenn die Dekontaminationsvorrichtung 50 ein Fluid-Luft-Gemisch so zerstäuben kann, dass ein möglichst kleiner Tropfendurchmesser erreicht wird, da eine optimierte Ausbringung angestrebt ist. Weiter sollte sie vorteilhaft so ausgelegt sein, dass die Funktionalität auch unter Mikrogravitations-Bedingungen sichergestellt ist. Dies gilt besonders für die Fluidförderung.

Für einen robusten Aufbau ist es bevorzugt, eine Dekontaminationsvorrichtung 50 zu verwenden, die auf ihre wesentlichen Funktionen reduziert ist.

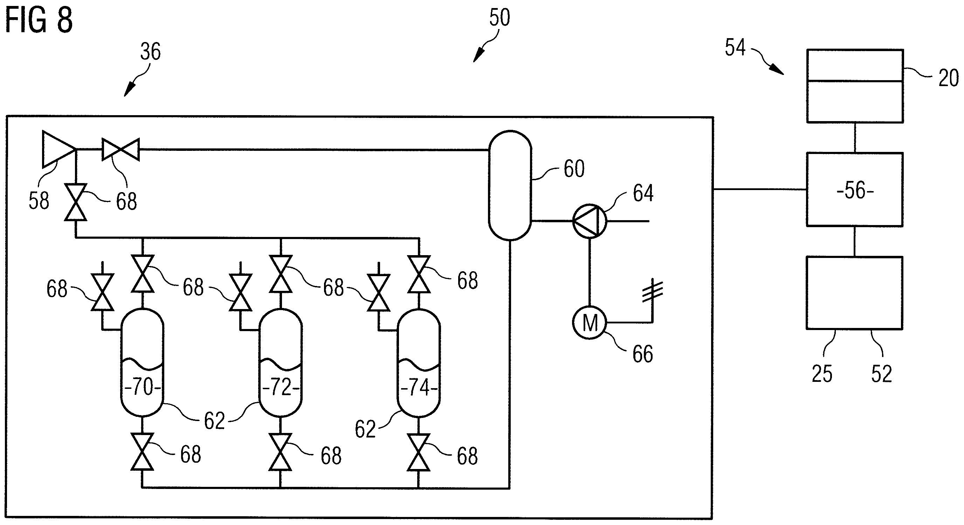

Fig. 8 zeigt eine solche Dekontaminationsvorrichtung 50.

Die Dekontaminationsvorrichtung 50 weist eine Identifizierungseinrichtung 52 und die Verteileinrichtung 36 auf. Weiter umfasst sie eine Auswahleinrichtung 54, um nach Identifikation einer Kontaminationsspezies 14 das passende Dekontaminationsmittel 16 aus einer Datenbank 20 auszuwählen. Die einzelnen Bauteile der Dekontaminationsvorrichtung 50 sind über eine Steuereinrichtung 56 gesteuert.

Die Verteileinrichtung 36 weist eine Zweikomponentendüse 58, einen Drucklufttank 60 mit einer Kapazität von 0,5 bis 20 bar, mehrere Behälter, insbesondere Membranbehälter 62, und einen Kompressor 64 auf.

Über den Kompressor 64 erfolgt die Luftzufuhr zu dem Drucklufttank 60. Weiter weist die Verteileinrichtung 36 auch einen Antrieb 66 mit Anschluss zum Anschließen an eine Energiequelle auf.

Der Drucklufttank 60 versorgt sowohl die Zweikomponentendüse 58 als auch die Membranbehälter 62 mit Druckluft, wobei die Mengen über Ventile 68 geregelt werden können. In einem der Membranbehälter 62 befindet sich eine Bakteriophagenlösung 70 (etwa 0,1 bis 20 Liter, abhängig von der Größe des Habitats 12). In einem anderen Membranbehälter 62 befindet sich eine chemische Lösung 72 (etwa 0,1 bis 20 Liter) und in einem weiteren Membranbehälter 62 Wasser 74 zur Reinigung (etwa 0,1 bis 20 Liter).

Da gerade die Zerstäubung von einigen Parametern abhängig ist, z.B. Fluidtemperatur, Geschwindigkeit, Pulsationsverhalten, Beladung (Verhältnis Fluid/Treibgas) und Geometrie der Dispergierdüse, und weiterhin die Applikationsmöglichkeit besteht, die Dekontaminationsvorrichtung 50 im Mikrogravitations-Bereich einzusetzen, wurden in Anlehnung an die Treibstoffförderung in Satelliten oder Trägerraketen Membranbehälter 62 als Fluidtanks vorgesehen werden. Diese ermöglichen nicht nur einen pulsationsfreien Vorschub des Fluids, sondern sind zusätzlich in der Lage, das Fluid schwerkraftunabhängig dosiert abzugeben.

Der Drucklufttank 60, dessen Kapazität in Druckbereichen zwischen 0,5 und 20 bar liegt, wird je nach Druckfall durch einen Kompressor 64 bzw. Verdichter auf Versorgungsstand gehalten. Um eine hohe Tropfengüte zu realisieren, ist ein pulsationsfreies Verhalten der Fluide bevorzugt. Pulsationsfreiheit wird an dieser Stelle durch die gleichmäßige Abgabe der Druckluft an die Düse 58, welche über ein Ventil 68 geregelt werden kann, realisiert. Die sekundäre Aufgabe des Druckluftspeichers 60 ist die individuelle Versorgung der Membrantanks 62 mit Druckluft, sodass diese einen konstanten Vorschub leisten können. Da auch hier das Vorschubgas pulsationsfrei geliefert wird, dient der Druckluftspeicher 60 im gesamten System der Bereitstellung eines pulsationsfreien Fluidverhaltens.

Es können mehrere Membrantanks 62 vorgesehen werden, um einen raschen Wechsel zwischen Bakteriophagen-Lösung 70 und chemischem Dekontaminationsmittel 16 zu ermöglichen.

Um eine gewollte Verteilung von Bakteriophagen 21 in einer anderen Lösung zu verhindern, wird ein Wassertank, der natürlich auch Reinigungszusätze aufweisen kann, vorgesehen. Ebenso ist bevorzugt, wenn Reste von aggressiven chemischen Dekontaminationsmitteln 16 in der Fluidleitung vermieden werden, um Bakteriophagen 21 nicht vorzeitig zu inaktivieren oder die eingesetzten Materialien nicht unnötig hoher Sauerstoff- bzw. Säurekorrosion auszusetzen.

Mit dem Wassertank und der entsprechenden Verschaltung können alle Membranbehälter 62 inkl. Leitungen gereinigt werden, sodass ein Einsatz mit einer neuen sensiblen Bakteriophagen-Lösung 70 durchgeführt werden kann.

Erfahrungen im komplizierten Zusammenspiel zwischen Identifikationseinheit und Dekontaminationseinheit können an einem Habitat-ähnlichen Versuchsaufbau gewonnen werden.

Der Habitat-Versuchsstand, im Folgenden vereinfacht "Habitat" 12 genannt, sollte bevorzugt die folgenden Anforderungen erfüllen:
- Das Habitat 12 ist bevorzugt ein abgeschlossenes System;
- Treten größere Temperaturunterschiede an der Habitathülle 76 auf, so sollte bevorzugt eine entsprechende Dämmung vorgesehen werden, um Kondensateffekte an der Habitat-Wandinnenseite vorerst zu vermeiden;
- Vorteilhaft besteht die Möglichkeit, die Habitatraumluft über ein klimatechnisches Versorgungssystem zu konditionieren. Dies betrifft vorrangig die Parameter Lufttemperatur, Luftfeuchte, Luftgeschwindigkeit;
- Weiterhin besteht bevorzugt die Möglichkeit, eine Raumluftumwälzung zu realisieren, um die Abhängigkeit der Aerosolverteilung anhand der Raumluft darstellen zu können;
- Das Habitat 12 verfügt bevorzugt über geeignete Messtechnik, um die oben genannten Parameter erfassen zu können;
- Das Habitat 12 verfügt vorteilhaft über Hindernisaufbauten 78, um "schlecht zugängliche" Bereiche zu simulieren;
- Die Materialen des Habitats 12 weisen bevorzugt eine Verträglichkeit gegenüber den klassischen chemischen Dekontaminationsmitteln 16, insbesondere Peressigsäure 34, auf;
- Zwischen den einzelnen Versuchsreihen wird bevorzugt eine "Nullung" der CFUs 80 durchgeführt, um wissenschaftlich belastbare Daten zu erhalten;
- Das Habitat 12 wird vorteilhaft unter den entsprechenden Sicherheitskriterien in einem geschützten Labor betrieben.

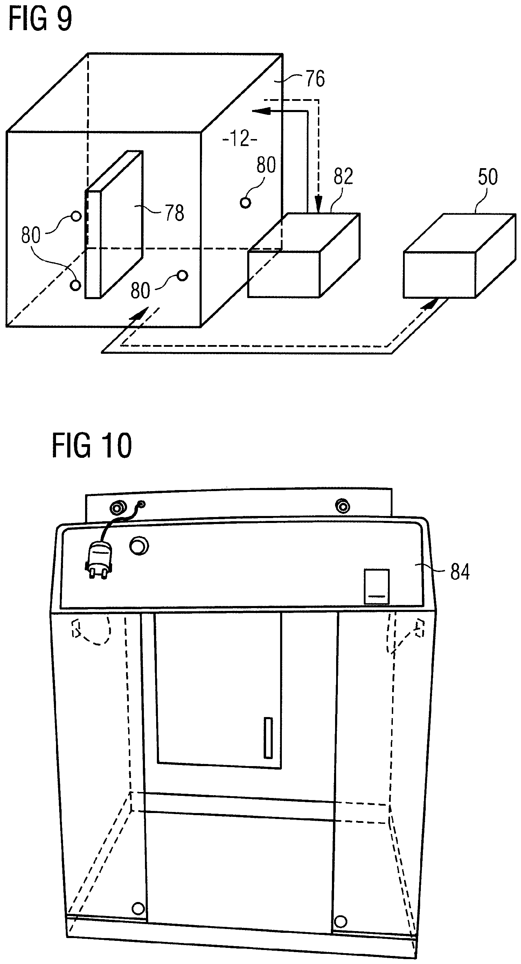

Ein vorteilhafter Bestandteil eines möglichen Versuchaufbaus, der in Fig. 9 dargestellt ist, sind Klimasysteme 82 zur Raumluftkonditionierung. Beide Systeme sind aufeinander abgestimmt. Eine isolierte Betrachtung beider Systeme wird nicht zu einer optimierten Systemanwendung mit minimalen Umwelteinflüssen führen. Gerade eine gezielte Zerstäubung eines Fluids kann je nach Raumgröße und Fluidmenge Auswirkungen auf die Raumluft ausüben. Im gleichen Maße kann eine angepasste Raumluft Feuchtelasten mindern oder gar vermeiden, sowie eine optimierte Ausbringung fördern.

Im geschlossenen System, dem Habitat 12, werden unterschiedliche mikrobiologische Proben, die CFUs 80, an unterschiedlichen Orten ausgebracht. Bevor jedoch diese ausgebracht werden, wird das Habitat 12 vollständig sterilisiert, um wissenschaftlich verwendbare Werte zu generieren.

Die ausgebrachten Proben können nun bei unterschiedlichen Atmosphäre-Bedingungen beobachtet werden.

Die wichtigsten Messparameter im Überblick sind:
- Habitatparameter;
- Lufttemperatur;
- Absolute und relative Luftfeuchte, Luftgeschwindigkeit;
- Luftwechselrate;
- Partikelverteilung/Tropfengröße,
- pH-Wert im Aerosol und auf Oberflächen bei chemischer Dekontamination;
- Mikrobiologische Parameter/Anzahl der CFUs 80;
- Veränderungen z.B. der Stoffwechselendprodukte (Messsignal für Identifikationssystem-Datenbank vor und nach Bakteriophageninfektion);
- Dauer bis die mikrobielle Belastung unter einen definierten Grenzwert gefallen ist;
- Zerstäuber;
- Fluidtemperaturen;
- Druckverhältnisse und Druckverluste, Beladungsverhältnis.

Für eine Durchführung werden drei Versuchsphasen mit unterschiedlichem Kenntnisstand vorgeschlagen:
- Phase I: kurzer Vorversuch mit abgeschlossener Box und Bakteriophagenaerosol;
- Phase II: umfangreicher Habitat-Versuchsstand mit chemischem Fluid-Aerosol;
- Phase III: umfangreicher Habitat-Versuch mit Bakteriophagen 21 als Fluid-oder Feststoff-Aerosol.

Innerhalb der Phase I ist ein kurzer Vorversuch bzgl. der prinzipiellen Bakteriophagen-Aerosolzerstäubung denkbar. Eine geschlossene Box 84, wie in Fig. 10 gezeigt, ist für diese ersten Tests ausreichend.

In dieser Phase I ist auch keine aufwendige Dekontaminationsvorrichtung 50 notwendig, sondern es kann auch der bereits erwähnte Zerstäuber benutzt werden.

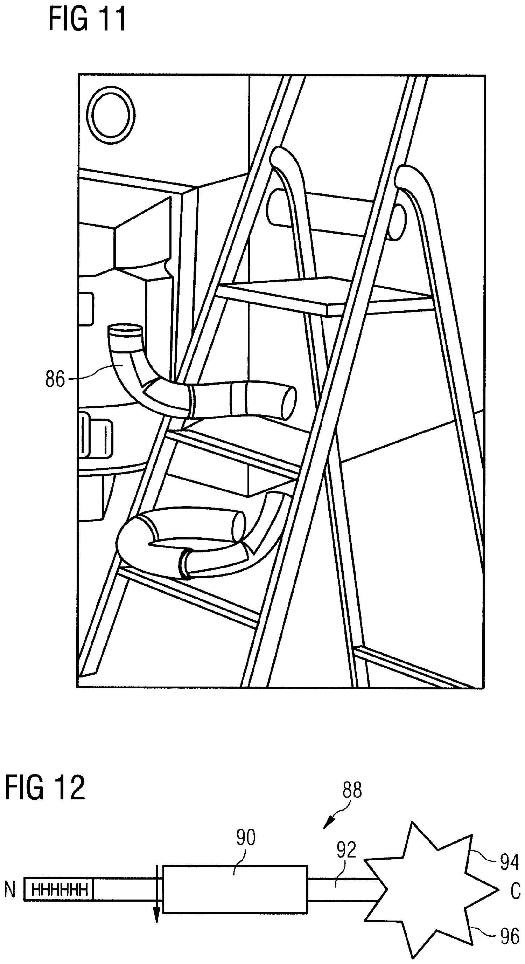

Ein Aufbau mit z.B. geschlitzten PET-Rohren 86, wie in Fig. 11 gezeigt, die das Vordringen des Aerosols aufzeigen sollen, gegebenenfalls auch in Kombination mit Feuchteindikatoren, kann die angestrebte Funktionsprüfung durchaus stützen. Nach einer "biologischen Nullung", z.B. durch UV-Strahlung oder chemische Dekontaminationsmittel 16, ist eine einfache Beaufschlagung von Bakterien 10 mit einem Bakteriophagenaerosol ausreichend, um erste prinzipielle Aussagen über die aerosolgestützte Bakteriophagendesinfektion zu bekommen.

Ist die grundsätzliche Funktionalität nachgewiesen, dann wird empfohlen, in Phase II einen größeren Habitat-ähnlicheren Messstand zu realisieren. Hierbei wird bevorzugt auf die Skalierung des Messtandes Wert gelegt. Mikroorganismen verbreiten sich über Raumluftströmungen. Um zu überprüfen, ob Bakteriophagen 21 den Bakterien 10 in ihrer Verbreitung nachfolgen können, weist das Versuchshabitat vorzugsweise eine gewisse Mindestgröße auf, um der natürlichen Raumluftströmung eine Umwälzung zu ermöglichen. Ebenso ermöglicht eine breite und gleichmäßige Raumluftströmung die gleichmäßige Verteilung des ausgebrachten Aerosols. Die in der Zweistoffdüse gebildeten Tropfen benötigen eine Mindestraumgröße, um in der Raumluft teilzuverdampfen und ein gleichmäßiges "Schwebeverhalten" zu etablieren.

Die Phase II beschäftigt sich zunächst mit der chemischen Dekontamination. Hier werden Erfahrungen gesammelt, wie die Dekontaminationsvorrichtung 50 mit dem chemischen Dekontaminationsmittel 16 arbeitet. Von besonderem Interesse ist die Einstellung der optimalen Parameter der Dekontaminationsvorrichtung 50.

Weiterhin ist die Abhängigkeit der chemischen Wirkstoffmenge von der Raumlufttemperatur und Raumluftfeuchte von Interesse, da hier Möglichkeiten bestehen, die Fluid- und Wirkstoffmenge zu reduzieren, um somit Nachteile der chemischen Dekontamination abzuschwächen, insbesondere die Aggressivität gegenüber vielen Materialien. Die zu messenden Temperaturen und Feuchten werden vorteilhaft in unterschiedlichen Höhen innerhalb des Habitats 12 ermittelt, sowie an unterschiedlichen Orten.

Der Versuch wird so gestaltet, dass nach einer vorherigen "biologischen Nullung" zunächst Bakterien 10 und Pilze ausgebracht werden. Diese werden an unterschiedlichen Orten und Höhen ausgebracht. An dieser Stelle ist es denkbar, das bakterielle Wachstum zu visualisieren, um die Ausbreitung sichtbar zu machen. Dies ist aus dem Grund interessant, da sich während eines späteren Einsatzes von Bakteriophagen 21 ein ähnliches Ausbreitungsverhalten darstellen wird.

Sind die Mirkoorganismen ausgebracht und die Raumluft wie gewünscht konditioniert, kann das chemische beladene Aerosol 46 im Raum und über die Strömung verteilt werden. Nach einer gewissen Wartezeit können die Mikrooganismen eingesammelt werden und im Labor näher untersucht werden.

Hier sind vor allem die Reduktionsstufen interessant, also um welchen "log-Faktor" die CFUs 80 reduziert wurden.

Anschließend wird das Habitat 12 vollständig gereinigt und sterilisiert, um für weitere Versuche zur Verfügung zu stehen.

Nach dem Nachweis der chemischen Dekontamination mit der Dekontaminationsvorrichtung 50 und Optimierung der Parameter kann in Phase III der Einsatz mit einer Bakteriophagensuspension erfolgen, oder je nach Abwandlung der vorgeschlagenen Dekontaminationsvorrichtung 50 eine trockene Feststoffzerstäubung mit Bakteriophagenpulver.

Der Versuch wird analog zu dem Versuch in Phase II durchgeführt.

Dies betrifft die "biologische Nullung", die Raumluftkonditionierung und die Probenausbringung. Anschließend werden die Bakteriophagen 21 innerhalb einer Lösung zerstäubt ausgebracht oder in Pulverform mittels eines Feststoff-Aerosolgenerators.

Nach der Ausbringung der Bakteriophagen 21 werden in gewissen Abständen Proben von unterschiedlichen Orten entnommen, um das Expansionsverhalten der Bakteriophagen 21 nachzuweisen und erste Abschätzungen über mögliche Wirkdauern zu treffen. Auch könnte man mittels fluoreszierenden Bakteriophagen-DNA-Markern eine Visualisierung der Bakteriophagenausbreitung realisieren, um damit die Geschwindigkeit und das Vordringen der Bakteriophagen 21 in animierter Form, z.B. über Kamerabilder, zeigen zu können. Innerhalb von Phase III können unterschiedliche Bakterienstämme 22 und Arten ausgebracht werden. Bakteriophagen 21 werden jedoch nur für einen bestimmten Stamm spezifisch ausgewählt.

Nach der Aerosolbeaufschlagung ist der bakteriophagenspezifische Stamm nicht mehr vorhanden oder nur noch in sehr geringen Mengen. Weitere Stämme können dagegen in ihrer Konzentration unverändert sein. Damit kann die Spezifität des Systems nachgewiesen werden.

Nach Phase II und III werden die abgetöteten Probenbehälter in Nährlösungen gegeben, um eine weitere Vermehrung und Wachstum von einer gegebenenfalls geringen Anzahl lebender Bakterien 10 anzuregen. Die mit Bakteriophagen 21 beaufschlagten Proben aus Phase III weisen jedoch kein größeres Bakterienwachstum auf, was wiederum den Beweis erbringt, dass durch die Bakteriophagen 21-gestützte Aerosoldesinfektion eine nachhaltige Wirkung aufrecht erhalten werden kann (balanciertes Gleichgewicht zwischen Populationen von Bakterien 10 und Bakteriophagen 21).

In Phase II und III wird das Identifikationssystem 25, z.B. die E-Nose, zusätzlich zu Untersuchungen im Labor zur Identifikation von Mikroorganismen eingesetzt. Hier werden Erfahrungen gewonnen, die den Umgang mit dem Identifikationssystem 25 weiter verbessern und die dazugehörige Datenbank 20 wird entsprechend erweitert.

Sind die mikrobiellen Belastungen in allen drei Phasen erfolgreich reduziert, dann ist der Beweis erbracht, dass der Testaufbau in Kombination mit der spezifischen Bakteriophagenauswahl biologisch, spezifisch und nachhaltig wirkt. Weiterhin kann zusätzlich eine klassische, chemische Dekontamination durchführt werden, um eine erhöhte Sicherheit zu garantieren. Des Weiteren dienen diese Versuchsreihen dazu, Erfahrungen im Systemumgang zu erwerben.

Das Konzept der mikrobiellen Identifikation und spezifischen Bakteriophagen 21-gestützten Dekontamination verfolgt einen innovativen Ansatz.

Ein vorhandenes Ökosystem (innerhalb eines Habitats 12) wird spezifisch und biologisch so eingestellt, dass jene Bakterienstämme 22, die für den Menschen, für Tiere oder Nutzpflanzen schädlich sein können, reduziert oder eliminiert werden. Es besteht ein Gleichgewicht in der Population zwischen den Bakterien 10 und den zugehörigen spezifischen Bakteriophagen 21. Bei der Dekontamination werden aber nicht alle vorhandenen Mikroorganismen im Habitat 12 vernichtet, sondern nur jene, die unerwünscht sind. Sollte der spezifische Ansatz nicht zum gewünschten Erfolg führen, z.B. weil (noch) keine isolierten Bakteriophagen 21 (kommerziell) erhältlich sind, verbleibt die klassische chemische Methode als weiteres Dekontaminationssystem. Somit kommt eine umfassende Strategie zum Einsatz, die Innovation und neue Ideen zur Anwendung bringt, aber gleichzeitig bewährte Technologien mit berücksichtigt.

Da nicht nur die Identifikationstechnik, sondern auch die Dekontaminationssysteme sehr komplexen mikrobiologischen und externen Randbedingungen ausgesetzt sind, liegt das eigentliche Know-how neben der technischen Applikation auch im Verständnis der diversen Randbedingungen und der Adaption auf sehr spezielle Anwendungsfälle. Dazu dient der Aufbau einer umfassenden mikrobiologischen Datenbank 20, die die rasche Identifikation der mikrobiologischen Belastung ermöglicht, da das Erkennen eines Mirkoorganismus an Randbedingungen, wie z.B. Bakterienstamm 22, Alter, Größe, Nährboden, Temperatur etc. geknüpft ist.

Mit dem skizzierten Versuchskonzept und dem vorgeschlagenen Test-Setup können jene Nachweise erbracht werden, die in der Diskussion mit potentiellen Kunden von entscheidender Bedeutung sein können.

In Zeiten von multi-resistenten Bakterien 10, immer höheren Wohndichten, sowie Globalisierung in wirtschaftlicher Hinsicht aber auch was das Reiseverhalten der Menschen anbetrifft, ist das Verfahren eine zu berücksichtigende Möglichkeit, zukünftigen Ausbreitungen von Bakterien 10 entgegenzuwirken. Das Verfahren kann die Reaktionsgeschwindigkeit im Wettlauf Bakterie-Mensch für den Menschen erhöhen und bietet somit eine Möglichkeit, angemessen zu reagieren.

Als Alternative zur Verwendung von ganzen Bakteriophagen 21 können auch Fusionsproteine 88 (dargestellt in Fig. 12) verwendet werden, die durch Zusammenbau eines Bakteriophagenproteins 90, eines Linkerproteins 92 und eines Markerproteins 94 in Form eines lytischen Proteins 96 erzeugt werden.

Die Herstellung solcher Fusionsproteine 88 kann der deutschen Patentanmeldung DE 10 2013 106 462.0 mit dem Titel "Detektionsverfahren zur Detektion von Bakterien, Verfahren zur Herstellung von Fusionsproteinen und Fusionsprotein", die als Anlage beigefügt ist, entnommen werden.

### Bezugszeichenliste:

- 10: Bakterium
- 12: Habitat
- 14: Kontaminationsspezies
- 16: Dekontaminationsmittel
- 18: Sensorarray
- 20: Dekontaminations-Datenbank
- 21: Bakteriophagen
- 22: Bakterienstamm
- 24: Bakterienart
- 25: Identifikationssystem
- 26: Wasserstoffperoxid
- 28: Aceytylhydroperoxid
- 30: Katalyseschutzschild
- 32: Aktivsauerstoff
- 34: Peressigsäuremolekül
- 35: Dekontaminationszustand
- 36: Verteileinrichtung
- 38: Vorschubkolben
- 40: Staubbehälter
- 42: Bürste
- 44: Luft
- 46: Aerosol
- 48: Zweistoffdüse
- 50: Dekontaminationsvorrichtung
- 52: Identifizierungseinrichtung
- 54: Auswahleinrichtung
- 56: Steuereinrichtung
- 58: Zweikomponentendüse
- 60: Drucklufttank
- 62: Membranbehälter
- 64: Kompressor
- 66: Antrieb mit Anschluss
- 68: Ventil
- 70: Bakteriophagenlösung
- 72: chemische Lösung
- 74: Wasser
- 76: Habitathülle
- 78: Hindernisaufbau
- 80: CFU
- 82: Klimasystem
- 84: Box
- 86: PET-Rohr
- 88: Fusionsprotein
- 90: Bakteriophagenprotein
- 92: Linkerprotein
- 94: Markerprotein
- 96: lytisches Protein

## Patentansprüche

1. Verfahren zur Dekontamination eines Habitats (12), aufweisend die Schritte
a) spezifisches Identifizieren wenigstens einer Kontaminationsspezies (14) in dem Habitat (12);
b) Auswählen eines auf die identifizierte Kontaminationsspezies (14) spezifisch wirksamen Dekontaminationsmittels (16), wobei biologische und chemische Dekontaminationsmittel (16) bereitgestellt werden, wobei als biologische Dekontaminationsmittel (16) spezifische Bakteriophagen (21) und/oder spezifische bakteriolytische Proteine (96) bereitgestellt werden und wobei als chemische Dekontaminationsmittel (16) chemische Dekontaminationsmittel (16) aus der Gruppe Aldehyde, Aldehydderivate, insbesondere Formaldehydderivate, Perverbindungen, insbesondere Peressigsäure, Phenole, Phenolderivate, Alkohole, Halogene, Halogenverbindungen, wasserstoffperoxidbasierte Dekontaminationsmittel, insbesondere Acetylhydroperoxid, bereitgestellt werden;
c) Verteilen des Dekontaminationsmittels (16) in dem zu dekontaminierenden Habitat (12);
d) Analyse des Dekontaminationszustandes (35) in dem Habitat (12),
wobei in Schritt b) bei mehreren spezifisch wirksamen Dekontaminationsmitteln (16) biologische Dekontaminationsmittel (16) ausgewählt werden.

2. Verfahren nach Anspruch 1,
**dadurch gekennzeichnet, dass** die Kontaminationsspezies (14) unter Verwendung eines Identifikationssystems (25) identifiziert wird, wobei biologische und/oder chemische Kontaminationsspezies (14), insbesondere eine Bakterienart (24) und/oder ein Bakterienstamm (22), identifiziert werden.

3. Verfahren nach einem der Ansprüche 1 oder 2,
**dadurch gekennzeichnet, dass** in Schritt b) das Dekontaminationsmittel (16) durch Vergleich der in Schritt a) identifizierten Kontaminationsspezies (14) mit einer Dekontaminationsmittel-Datenbank (20) ausgewählt wird, in der der Kontaminationsspezies (14) wenigstens ein spezifisch wirksames Dekontaminationsmittel (16) zugeordnet ist.

4. Verfahren nach einem der Ansprüche 1 bis 3,
**dadurch gekennzeichnet, dass** als spezifische bakteriolytische Proteine (96) spezifische bakteriolytische Fusionsproteine (88) bereitgestellt werden.

5. Verfahren nach Anspruch 4,
**dadurch gekennzeichnet, dass** die Bakteriophagen (21) genetisch derart modifiziert werden, dass Transduktionsgene zum Transferieren bakterieller Virulenzfaktorgenen in das Bakteriophagen (21) inaktiviert werden, insbesondere durch spezifische Mutation, mehr insbesondere durch Punktmutation, Deletion, Insertion, Inversion und/oder Rasterschubmutation.

6. Verfahren nach einem der Ansprüche 4 oder 5,
**dadurch gekennzeichnet, dass** Fusionsproteine (88) mit den folgenden Schritten hergestellt werden:
A) Klonieren eines Fusionsproteingens;
B) Transformieren des Fusionsproteingens in einen niederen prokaryotischen oder eukaryotischen Organismus;
C) Exprimieren von Fusionsproteinen (88) in dem Organismus;
D) Reinigen der Fusionsproteine (88),
wobei in Schritt A) ein Bakteriophagenprotein-Gen, ein Linkerprotein-Gen und ein lytisches Protein-Gen ligiert werden.

7. Verfahren nach einem der Ansprüche 4 bis 6,
**dadurch gekennzeichnet, dass** die Bakteriophagen (21) und/oder die Fusionsproteine (88) stabilisiert werden, insbesondere gegen Denaturierung, hervorgerufen durch physikalische, biologische und/oder chemische Einflüssewobei als Stabilisierungsmethode eine oder mehrere Stabilisierungsmethoden aus der Gruppe
- Vakuumtrocknung;
- Gefriertrocknung;
- Lyophilisation;
- Sublimationstrocknung;
- Zugabe von Stabilisatoren, insbesondere von Sacchariden, Sucrosen, Glycerolen, Polyolen, Polyethylenglycolen, Aminosäuren, Methylaminen und/oder anorganischen Salzen;
- Glykosilierung;
- PEGylierung, insbesondere durch unvollständiges Umhüllen des Bakteriophagen (21) mit Polyethylenglycolmolekülen unter freiem Verbleiben der kontraktilen Scheide des Bakteriophagen (21)
ausgewählt wird.

8. Verfahren nach einem der Ansprüche 1 bis 7,
**dadurch gekennzeichnet, dass** unterschiedliche Bakteriophagenstämme und/oder eine Mischung aus Bakteriophagen (21) und bakteriolytischen Proteinen (96), insbesondere Fusionsproteinen (88), bereitgestellt werden.

9. Verfahren nach einem der Ansprüche 1 bis 8,
**dadurch gekennzeichnet, dass** vor Schritt d) eine Kontaminationskonzentrationsgrenze definiert wird und in Schritt d) der Dekontaminationszustand (35) in dem Habitat (12) mit der Kontaminationskonzentrationsgrenze verglichen wird, wobei bei Überschreiten der Kontaminationskonzentrationsgrenze die Schritte a) bis d) wiederholt werden.

10. Verfahren nach Anspruch 9,
**dadurch gekennzeichnet, dass** bei Wiederholung der Schritte a) bis d) das zuvor ausgewählte Dekontaminationsmittel (16) oder ein anderes auf die identifizierte Kontaminationsspezies (14) spezifisch wirksames Dekontaminationsmittel (16) ausgewählt wird.
Airbus Defence and Space GmbH, et al

11. Verfahren nach einem der Ansprüche 1 bis 10,
**dadurch gekennzeichnet, dass** das Dekontaminationsmittel (16) in Schritt c) in trockenem Zustand oder in Lösung (70), insbesondere in Pufferlösung, oder als Suspension verteilt wird.

12. Verfahren nach einem der Ansprüche 1 bis 11,
**dadurch gekennzeichnet, dass** nach der Dekontamination des Habitats (12) als Dekontaminationsmittel (16) verwendete Bakteriophagen (21) aus dem Habitat (12) entfernt werden, insbesondere unter Verwendung wenigstens eines Filters, mehr insbesondere unter Verwendung wenigstens eines HEPA-Filters.

13. Dekontaminationsvorrichtung (50) zur Dekontamination eines Habitats (12), aufweisend eine Identifizierungseinrichtung (52) zum Identifizieren wenigstens einer Kontaminationsspezies (14) in einem Habitat (12), eine Auswahleinrichtung (54) zum Auswählen eines auf die identifizierte Kontaminationsspezies (14) spezifisch wirksamen Dekontaminationsmittels (16), wobei die Auswahleinrichtung (54) eine Dekontaminations-Datenbank aufweist, in der der Kontaminationsspezies (14) wenigstens ein wirksames Dekontaminationsmittel (16) zugeordnet ist, und eine Verteileinrichtung (36) zum Verteilen des ausgewählten Dekontaminationsmittels (16) in dem Habitat (12),
**dadurch gekennzeichnet, dass** die Verteileinrichtung (36) eine Düse (58), einen Druckluftspeicher (60) und wenigstens einen Membranbehälter (62) aufweist, sowie eine Steuereinrichtung zum Steuern der einzelnen Bauteile zur Durchführung des Verfahrens gemäß Anspruch 1.

## Claims

1. A method for the decontamination of a habitat (12), comprising the steps of
a) specifically identifying at least one contamination species (14) in the habitat (12);
b) selecting a decontaminant (16) that is specifically effective against the identified contamination species (14), wherein biological and chemical decontaminants (16) are provided, wherein specific bacteriophages (21) and/or specific bacteriolytic proteins (96) are provided as biological decontaminants (16), and wherein chemical decontaminants (16) from the group including aldehydes, aldehyde derivatives, in particular formaldehyde derivatives, per compounds, in particular peracetic acid, phenols, phenol derivatives, alcohols, halogens, halogen compounds, hydrogen peroxide-based decontaminants, in particular acetyl hydroperoxide, are provided as chemical decontaminants (16);
c) distributing the decontaminant (16) in the habitat (12) to be decontaminated;
d) analyzing the decontamination state (35) in the habitat (12),
wherein
biological decontaminants (16) are selected in step b), given several specifically effective decontaminants (16).

2. The method according to claim 1,
**characterized in that** the contamination species (14) is identified using an identification system (25), with biological and/or chemical contamination species (14), particularly a bacterial species (24) and/or a bacterial strain (22), being identified.

3. The method according to any one of the claims 1 or 2,
**characterized in that**, in step b), the decontaminant (16) is selected by comparing the contamination species (14) identified in step a) with a decontaminant database (20) in which at least one specifically effective decontaminant (16) is associated with the contamination species (14).

4. The Method according to any one of the claims 1 to 3,
**characterized in that** specific bacteriolytic fusion proteins (88) are provided as specific bacteriolytic proteins (96).

5. The method according to claim 4,
**characterized in that** the bacteriophages (21) are genetically modified in such a way that transduction genes for transferring bacterial virulence factor genes into the bacteriophage (21) are deactivated, in particular by specific mutation, more particularly by point mutation, deletion, insertion, inversion and/or frameshift mutation.

6. The method according to any one of the claims 4 or 5,
**characterized in that** fusion proteins (88) are prepared by means of the following steps:
A) cloning a fusion protein gene;
B) transforming a fusion protein gene into a lower prokaryotic or eukaryotic organism;
C) expressing fusion proteins (88) in the organism;
D) purifying the fusion proteins (88),
wherein a bacteriophage protein gene, a linker protein gene and a lytic protein gene are ligated in step A).

7. The method according to any one of the claims 4 to 6,
**characterized in that** the bacteriophages (21) and/or the fusion proteins (88) are stabilized, in particular against denaturation caused by physical, biological and/or chemical influences, with one or more stabilization methods from the group including
- vacuum drying;
- freeze drying;
- lyophilization;
- sublimation drying;
- addition of stabilizers, in particular of saccharides, sucroses, glycerols, polyols, polyethylene glycols, amino acids, methylamines and/or inorganic salts;
- glycosylation;
- PEGylation, in particular by means of incomplete encapsulation of the bacteriophage (21) with polyethylene glycol molecules while the contractile sheath of the bacteriophage (21) remains free
being selected as the stabilization method.

8. The method according to any one of the claims 1 to 7,
**characterized in that** different bacteriophage strains and/or a mixture of bacteriophages (21) and bacteriolytic proteins (96), in particular fusion proteins (88), are provided.

9. The method according to any one of the claims 1 to 8,
**characterized in that** a contamination concentration limit is defined prior to step d), and the decontamination state (35) in the habitat (12) is compared with the contamination concentration limit in step d), with the steps a) to d) being repeated in the event the contamination concentration limit is exceeded.

10. The method according to claim 9,
**characterized in that**, in the event the steps a) to d) are repeated, the previously selected decontaminant (16) or a different decontaminant (16), which is specifically effective against the identified contamination species (14), is selected.

11. The method according to any one of the claims 1 to 10,
**characterized in that** the decontaminant (16) is distributed in a dry state or in solution (70), in particular in a buffer solution, or as a suspension in step c).

12. The method according to any one of the claims 1 to 11,
**characterized in that** bacteriophages (21) used as decontaminants (16) are removed from the habitat (12) subsequent to the decontamination of the habitat (12), in particular using at least one filter, more particularly using at least one HEPA filter.

13. A decontamination device (50) for the decontamination of a habitat (12) comprising an identification device (52) for identifying at least one contamination species (14) in a habitat (12), a selection device (54) for selecting a decontaminant (16) that is specifically effective against the identified contamination species (14), the selection device (54) having a decontamination database in which at least one effective decontaminant (16) is associated with the contamination species (14), and a distribution device (36) for distributing the selected decontaminant (16) in the habitat (12),
**characterized in that** the distribution device (36) has a nozzle (58), a compressed-air storage device (60) and at least one membrane vessel (62), and a control device for controlling the individual components for carrying out the method according to claim 1.

## Revendications

1. Procédé de décontamination d'un habitat (12) comprenant des étapes consistant à :
a) identifier de manière spécifique au moins une espèce contaminante (14) dans l'habitat (12),
b) sélectionner un agent de décontamination (16) spécifiquement efficace sur l'espèce contaminante identifiée (14), des agents de décontamination (16) biologiques et chimiques étant préparés, en tant qu'agent de décontamination biologique (16) des bactériophages spécifiques (21) et/ou des protéines bactériolytiques spécifiques (96) étant préparés, et en tant qu'agent de décontamination chimique (16) des agents de décontamination chimique (16) choisis dans le groupe composé par les aldéhydes, les dérivés d'aldéhydes, en particulier les dérivés de formaldéhyde, les dérivés peroxygénés, en particulier l'acide peracétique, les phénols, les dérivés de phénols, les alcools, les halogènes, les composés halogénés, les agents de décontamination à base de peroxide d'hydrogène, en particulier l'acétylhydropéroxide étant préparés,
c) répartir l'agent de décontamination (16) dans l'habitat (12),
d) analyser l'état de décontamination (35) dans l'habitat (12), procédé selon lequel dans l'étape b) en présence de plusieurs agents de décontamination (16) spécifiquement actifs, des agents de décontamination biologique (16) sont choisis.

2. Procédé conforme à la revendication 1,
**caractérisé en ce que**
les espèces contaminantes (14) sont identifiées en utilisant un système d'identification (25), des espèces contaminantes biologiques et/ou chimiques (14) en particulier une espèce bactérienne (24) et/ou une souche bactérienne (22) étant identifiées.

3. Procédé conforme à l'une des revendications 1 et 2,
**caractérisé en ce que**
lors de l'étape b) l'agent de décontamination (16) est sélectionné par comparaison de l'espèce contaminante (14) identifiée lors de l'étape a) avec une banque de données (20) d'agents de décontamination dans laquelle à l'espèce contaminante (14) est associé au moins un agent de décontamination (16) spécifiquement actif.

4. Procédé conforme à l'une des revendications 1 à 3,
**caractérisé en ce qu'**
en tant que protéine bactériolytique spécifique (96) des protéines de fusion bactériolytique spécifiques (88) sont préparées.

5. Procédé conforme à la revendication 4,
**caractérisé en ce que**
les bactériophages (21) sont génétiquement modifiés de sorte que des gènes de transduction permettant de transférer des gênes de facteur de virulence bactériens dans le bactériophage (21) soit inactivés, en particulier par mutation spécifique, et de façon plus préférentielle par mutation ponctuelle, délétion, insertion, inversion et/ou mutation avec décalage du cadre de lecture.

6. Procédé conforme à l'une des revendications 4 et 5,
**caractérisé en ce que**
des protéines de fusion (88) sont obtenues par la mise en oeuvre des étapes suivantes consistant à :
A) cloner un gène de protéine de fusion,
B) transformer le gène de protéine de fusion en un organisme eucaryote ou procaryote inférieur,
C) exprimer des protéines de fusion (88) dans l'organisme,
D) purifier les protéines de fusion (88),
procédé selon lequel dans l'étape A) un gêne de protéine bactériophage, un gêne de protéine de liaison et un gène de protéine lytique sont ligaturés.

7. Procédé conforme à l'une des revendications 4 à 6,
**caractérisé en ce que**
les bactériophages (21) et/ou les protéines de fusion (88) sont stabilisées en particulier pour empêcher leur dénaturation provoquée par influence physique, biologique et/ou chimique,
procédé selon lequel en tant que méthode de stabilisation on sélectionne au moins une méthode de stabilisation du groupe suivant :
- séchage sous vide,
- séchage par congélation,
- lyophilisation,
- séchage par sublimation,
- addition d'agents de stabilisation, en particulier de saccarides, de sucroses, de glycérols, de polyols, de polyéthylène glycols, d'acides aminés, de méthyl amides et/ou de sels inorganiques,
- glycolisation,
- PEGlycolisation en particulier par enrobage incomplet du bactériophage (21) par des molécules de polyéthylène glycol en laissant libres les fibres contractiles du bactériophage (21).

8. Procédé conforme à l'une des revendications 1 à 7,
**caractérisé en ce que**
l'on prépare différentes souches de bactériophages et/ou un mélange de bactériophages (21) et de protéines bactériolytiques (86), en particulier de protéines de fusion (88).

9. Procédé conforme à l'une des revendications 1 à 8,
**caractérisé en ce qu'**
avant l'étape d) on définit une limite de concentration de contamination et lors de l'étape d) on compare l'état de décontamination (35) dans l'habitat (12) avec la limite de concentation de contamination, et, en cas de dépassement de la limite de concentration de contamination les étapes a) à d) sont répétées.

10. Procédé conforme à la revendication 9,
**caractérisé en ce que**
lors de la répétition des étapes a) à d), l'agent de décontamination (16) préalablement sélectionné ou un autre agent de décontamination (16) spécifiquement actif sur l'espèce contaminante (14) identifiée est sélectionné.

11. Procédé conforme à l'une des revendications 1 à 10,
**caractérisé en ce que**,
lors de l'étape c) l'agent de décontamination (16) est distribué à l'état sec ou en solution (70), en particulier en solution tampon ou sous la forme de suspension.

12. Procédé conforme à l'une des revendications 1 à 11,
**caractérisé en ce que**,
après la décontamination de l'habitat (12), les bactériophages (21) utilisés en tant qu'agent de décontamination (16) sont éliminés de l'habitat (12), en particulier par utilisation d'au moins un filtre et de façon particulièrement préférentielle, par utilisation d'au moins un filtre HEPA.

13. Dispositif de décontamination (50) destiné à permettre de décontaminer un habitat (12) comportant un dispositif d'identification (52) permettant d'identifier au moins une espèce contaminante (14) dans un habitat (12), un dispositif de sélection (54) permettant de sélectionner un agent de décontamination (16) spécifiquement actif sur l'espèce contaminante (16) identifiée, le dispositif de sélection (54) comprenant une banque de données de décontamination dans laquelle à l'espèce contaminante (14) est associé au moins un agent de décontamination actif (16), et un dispositif de répartition (36) permettant de répartir l'agent de décontamination (16) sélectionné dans l'habitat (12),
**caractérisé en ce que**
le dispositif de répartition (36) comprend une buse (58), un accumulateur d'air comprimé (60) et au moins un récipient à diaphragme (62), ainsi qu'un dispositif de commande permettant de commander les différents composants pour permettre la mise en oeuvre du procédé conforme à la revendication 1.
